# EUROPEAN PATENT SPECIFICATION

(11) **EP 3 973 904 B1**
(45) Date of publication and mention of the grant of the patent: **25.06.2025**
(21) Application number: 21198223.6
(22) Date of filing: 22.09.2021
(51) Int. Cl.: A61B 18/14

(54) **ELECTRODE SHORTING**
ELEKTRODENKURZSCHLUSS
COURT-CIRCUIT D'ÉLECTRODE

(30) Priority: 23.09.2020 US 202017029552
(43) Date of publication of application: 30.03.2022
(73) Proprietor: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL); BEECKLER, Christopher Thomas, Irwindale, 91706 (US); KEYES, Joseph Thomas, Irwindale, 91706 (US); HERRERA, Kevin Justin, Irwindale, 91706 (US)
(74) Representative: Carpmaels & Ransford LLP

(56) References cited:
- US-A1- 2013 304 062
- US-A1- 2016 331 459
- US-A1- 2017 000 365
- US-A1- 2017 042 449
- US-A1- 2017 312 024
- US-A1- 2018 085 160
- US-A1- 2020 069 364
- US-A1- 2020 205 890

## Description

### FIELD OF THE INVENTION

The present invention relates to medical devices, and in particular, but not exclusively to, ablation catheters.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied through the tip electrode(s) of the ablating catheter, and current flows through the media that surrounds it, i.e., blood and tissue, between the tip electrode(s) and an indifferent electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Irreversible electroporation (IRE) applies short electrical pulses that generate high enough electrical fields (typically greater than 450 Volts per centimeter) to irreversibly damage the cells. Non-thermal IRE may be used in treating different types of tumors and other unwanted tissue without causing thermal damage to surrounding tissue. Small electrodes are placed in proximity to target tissue to apply short electrical pulses. The pulses increase the resting transmembrane potential, so that nanopores form in the plasma membrane. When the electricity applied to the tissue is above the electric field threshold of the target tissue, the cells become permanently permeable from the formation of nanopores. As a result, the cells are unable to repair the damage and die due to a loss of homeostasis and the cells typically die by apoptosis.

IRE may be used for cardiac ablation as an alternative to other cardiac ablation techniques, e.g., radio-frequency (RF) cardiac ablation. IRE cardiac ablation is sometimes referred to as Pulse Field Ablation (PFA). As IRE is generally a low thermal technique, IRE may reduce the risk of collateral cell damage that is present with the other techniques. e.g., in RF cardiac ablation.

US Patent Publication No. 2020/0069364 to Salahieh, et al., describes cardiac tissue ablation catheters including an inflatable and flexible toroidal or spherically shaped balloon disposed at a distal region of an elongate member, a flexible circuit carried by an outer surface of the balloon, the flexible circuit including, a plurality of flexible branches conforming to the radially outer surface of the balloon, each of the plurality of flexible branches including a substrate, a conductive trace carried by the substrate, and an ablation electrode carried by the substrate, the ablation electrode in electrical communication with the conductive trace, and an elongate shaft comprising a guidewire lumen extending in the elongate member and extending from a proximal region of the inflatable balloon to distal region of the inflatable balloon and being disposed within the inflatable balloon, wherein a distal region of the elongate shaft is secured directly or indirectly to the distal region of the inflatable balloon.

US Patent No. 8,295,902 to Salahieh, et al., describes a tissue electrode assembly including a membrane configured to form an expandable, conformable body that is deployable in a patient. The assembly further includes a flexible circuit positioned on a surface of the membrane and comprising at least one base substrate layer, at least one insulating layer and at least one planar conducting layer. An electrically-conductive electrode covers at least a portion of the flexible circuit and a portion of the surface of the membrane not covered by the flexible circuit, wherein the electrically-conductive electrode is foldable upon itself with the membrane to a delivery conformation having a diameter suitable for minimally-invasive delivery of the assembly to the patient.

US Patent No. 10,470,682 to Deno, et al., describes a system for determining electrophysiological data comprising an electronic control unit configured to acquire electrophysiology signals from a plurality of electrodes of one or more catheters, select at least one clique of electrodes from the plurality of electrodes to determine a plurality of local E field data points, determine the location and orientation of the plurality of electrodes, process the electrophysiology signals from the at least one clique from a full set of bipole sub-cliques to derive the local E field data points associated with the at least one clique of electrodes, derive at least one orientation independent signal from the at least one clique of electrodes from the information content corresponding to weighted parts of electrogram signals, and display or output catheter orientation independent electrophysiologic information to a user or process.

US Patent Publication No. 2014/0200578 to Groff, et al., describes medical devices for ablating nerves perivascularly and methods for making and using the same. An example medical device may include an expandable frame slidably disposed within a catheter shaft. The expandable frame may be configured to shift between a collapsed configuration and an expanded configuration. One or more electrodes may be disposed on a surface of the expandable frame. The one or more electrodes may be disposed radially inward relative to the greatest radial extent of the expandable frame when the expandable frame is in the expanded configuration.

US Patent No. 6,004,269 to Crowley, et al., describes an acoustic imaging system for use within a heart has a catheter, an ultrasound device incorporated into the catheter, and an electrode mounted on the catheter. The ultrasound device directs ultrasonic signals toward an internal structure in the heart to create an ultrasonic image, and the electrode is arranged for electrical contact with the internal structure. A chemical ablation device mounted on the catheter ablates at least a portion of the internal structure by delivery of fluid to the internal structure. The ablation device may include a material that vibrates in response to electrical excitation, the ablation being at least assisted by vibration of the material. The ablation device may alternatively be a transducer incorporated into the catheter, arranged to convert electrical signals into radiation and to direct the radiation toward the internal structure. The electrode may be a sonolucent structure incorporated into the catheter.

US Patent Publication No. 2018/0125576 to Rubinstein, et al., describes a medical apparatus, used to acquire electrical activity of patient anatomy, which includes an elongated body and a tip portion coupled to the elongated body. The tip portion includes one or more inflatable sections. Each inflatable section has a plurality of electrodes disposed on one of: (i) an outer surface of the one or more inflatable sections; and (ii) an inner surface and the outer surface of the one or more inflatable sections. The one or more inflatable sections, when inflated, cause a portion of the plurality of electrodes to contact a surface of an organ and provide a pathway for physiological fluid to flow through the tip portion. In one embodiment, the tip portion is a tulip balloon tip portion. In another embodiment, the tip portion is an inflatable tip portion having one or more concentrically wound inflatable sections.

EP Patent Publication 3576657A1 describes electroporation systems and methods of energizing a catheter for delivering electroporation. A catheter for delivering electroporation includes a distal section and an electrode assembly. The distal section is configured to be positioned in a vein within a body. The vein defines a central axis. The electrode assembly is coupled to the distal section and includes a structure and a plurality of electrodes distributed thereabout. The structure is configured to at least partially contact the vein. Each of the electrodes is configured to be selectively energized to form a circumferential ring of energized electrodes that is concentric with the central axis of the vein.

US2017042449A1 discloses a system and method for local electrophysiological characterization of cardiac substrate using multi-electrode catheters. Disclosed is a system for determining electrophysiological data comprising an electronic control unit configured to acquire electrophysiology signals from a plurality of electrodes of one or more catheters, select at least one clique of electrodes from the plurality of electrodes to determine a plurality of local E field data points, determine the location and orientation of the plurality of electrodes, process the electrophysiology signals from the at least one clique from a full set of bipole subcliques to derive the local E field data points associated with the at least one clique of electrodes, derive at least one orientation independent signal from the at least one clique of electrodes from the information content corresponding to weighted parts of electrogram signals, and display or output catheter orientation independent electrophysiologic information to a user or process.

US2016331459A1describes a system for neuromodulation with a flexible catheter comprising a proximal electrode and a distal therapeutic assembly comprising multiple electrodes which can have the same polarity.

### SUMMARY

The invention is defined in independent claim 1, further embodiments are described in the dependent claims.

Methods of surgery described here below are not claimed but are helpful for understanding the invention.

There is provided in accordance with an embodiment of the present invention, a medical system including a catheter configured to be inserted into a body part of a living subject, and including a deflectable element having a distal end, an expandable distal end assembly disposed at the distal end of the deflectable element, and including a plurality of assembly electrodes, and configured to expand from a collapsed form to an expanded deployed form, a proximal electrode disposed at the distal end of the deflectable element proximally to the expandable distal end assembly, and extending circumferentially around the deflectable element, at least one electrical connection configured to electrically connect together at least two of the assembly electrodes to act as a combined assembly electrode, and an ablation power generator configured to be connected to the catheter, and apply an electrical signal between the combined assembly electrode and a selected electrode.

Further in accordance with an embodiment of the present invention the selected electrode is the proximal electrode.

Still further in accordance with an embodiment of the present invention the at least one electrical connection permanently electrically connects together the at least two assembly electrodes to act as the combined assembly electrode.

Additionally, in accordance with an embodiment of the present invention the at least one electrical connection is configured to electrical connect together all of the assembly electrodes to act as the combined assembly electrode.

Moreover, in accordance with an embodiment of the present invention the at least one electrical connection permanently electrically connects together all of the assembly electrodes to act as the combined assembly electrode.

Further in accordance with an embodiment of the present invention the expandable distal end assembly includes at least one of an expandable basket including a plurality of splines, the electrodes being disposed on the splines, or an inflatable balloon with the electrodes disposed thereon.

Still further in accordance with an embodiment of the present invention the proximal electrode includes irrigation holes through which to irrigate the body part, the catheter also including an irrigation tube disposed in the deflectable element and configured to be in fluid communication with the irrigation holes of the proximal electrode.

Additionally, in accordance with an embodiment of the present invention the irrigation holes are disposed radially around the proximal electrode.

Moreover, in accordance with an embodiment of the present invention the irrigation holes are disposed longitudinally along the proximal electrode.

Further in accordance with an embodiment of the present invention the proximal electrode and the deflectable element define an annular hollow therebetween, the irrigation tube being coupled to transfer irrigation fluid into the hollow, the irrigation tube being in fluid communication with the irrigation holes via the hollow.

Still further in accordance with an embodiment of the present invention, the system includes an irrigation reservoir configured to store irrigation fluid, and a pump configured to be connected to the irrigation reservoir and the catheter, and to pump the irrigation fluid from the irrigation reservoir through the irrigation holes via the irrigation tube.

Additionally, in accordance with an embodiment of the present invention the ablation power generator is configured to apply the electrical signal between the combined assembly electrode and the proximal electrode to perform electroporation of tissue of the body part.

Moreover, in accordance with an embodiment of the present invention, the system includes an irrigation tube disposed in the deflectable element and configured to deliver irrigation fluid into a region surrounded by the expandable distal end assembly.

Further in accordance with an embodiment of the present invention the proximal electrode has a maximum thickness measured perpendicular to the axis of the deflectable element of at least 0.05 mm and an inner diameter in the range of 2 mm to 6 mm.

According to the present invention the proximal electrode and the distal end of the deflectable element define an annular region therebetween, the catheter also including thermally conductive material disposed in the annular region, the thermally conductive material being formed from a different material than the proximal electrode.

There is also provided in accordance with another embodiment of the present invention, a medical system including a catheter configured to be inserted into a body part of a living subject, and including a deflectable element having a distal end, an expandable distal end assembly disposed at the distal end of the deflectable element, and including a plurality of assembly electrodes, and configured to expand from a collapsed form to an expanded deployed form, at least one electrical connection permanently electrically connecting together at least two of the assembly electrodes to act as a combined assembly electrode, and an ablation power generator configured to be connected to the catheter, and apply an electrical signal to the combined assembly electrode so as to ablate tissue of the body part.

Additionally, in accordance with an embodiment of the present invention the at least one electrical connection permanently electrically connects together all of the assembly electrodes to act as the combined assembly electrode.

Moreover, in accordance with an embodiment of the present invention the expandable distal end assembly includes at least one of an expandable basket including a plurality of splines, the electrodes being disposed on the splines, or an inflatable balloon with the electrodes disposed thereon.

Further in accordance with an embodiment of the present invention the ablation power generator is configured to apply the electrical signal to the combined assembly electrode so as to perform electroporation of tissue of the body part.

### BRIEF DESCRIPTION OF THE DRAWINGS

The present invention will be understood from the following detailed description, taken in conjunction with the drawings in which:
Fig. 1 is a schematic view of a medical system constructed and operative in accordance with an embodiment of the present invention;
Fig. 2 is a schematic view of a catheter in a deployed form constructed and operative in accordance with an embodiment of the present invention;
Fig. 3 is a schematic view of the distal end of the catheter of Fig. 2 in a collapsed form;
Fig. 4A is a cross-sectional view of the distal end of the catheter of Fig. 2;
Fig. 4B is a more detailed cross-sectional view of the distal end of the catheter inside block B of Fig. 4A;
Fig. 5A is a cross-sectional view of the catheter of Fig. 2 along line A:A;
Fig. 5B is a cross-sectional view of the catheter of Fig. 2 along line B:B;
Fig. 6 is a schematic view of a catheter in a deployed form constructed and operative in accordance with an alternative embodiment of the present invention;
Fig. 7 is a cross-sectional view of the catheter of Fig. 6 along line C:C;
Fig. 8 is a cross-sectional view of the catheter of Fig. 6 along line C:C constructed and operative in accordance with another alternative embodiment of the present invention;
Fig. 9 is a schematic view of a catheter in a deployed form constructed and operative in accordance with yet another alternative embodiment of the present invention; and
Fig. 10 is a schematic view of electrode connections in the catheter of Fig. 2.

### DESCRIPTION OF EXAMPLE EMBODIMENTS

### OVERVIEW

A balloon catheter or another catheter with an expandable distal end assembly such as a basket catheter may include electrodes on the distal end assembly that may be used for ablation, such as RF ablation or IRE ablation. In order to ablate a large area, a physician may need to reposition the catheter in order to adequately cover the area. This is time consuming.

Embodiments of the present invention solve the above problem by electrically connecting some or all of the electrodes of a distal end assembly of a catheter to act as a combined assembly electrode. An ablation power generator, connected to the catheter, applies an electrical signal to the combined assembly electrode to perform ablation (e.g., electroporation) of the tissue of the body part.

A return electrode may be used so that the ablation current is applied between the combined assembly electrode of the distal end assembly electrodes and a return electrode. In some cases, when the return electrode is one of the electrodes on the distal end assembly or in the middle of the distal end assembly, the ablation current may avoid travelling through the tissue thereby reducing the efficacy of the ablation current. Therefore, in some embodiments, the catheter includes a proximal electrode placed proximally to the distal end assembly. The ablation power generator applies an electrical signal between the combined assembly electrode and the proximal electrode to perform ablation (e.g., electroporation) of tissue of the body part.

Placing the return electrode proximally to the expandable distal end assembly helps prevent the ablation current from travelling inside the distal end assembly. However, due to the concentration of the ablation energy at the proximal return electrode, the proximal return electrode may overheat or cause charring of tissue.

Embodiments of the present invention solve the above problems by providing an irrigated proximal electrode, which is placed at the distal end of a deflectable element of the catheter, proximally to the distal end assembly. The proximal electrode extends circumferentially around the deflectable element, and includes irrigation holes through which to irrigate the body part to prevent overheating and charring. An irrigation tube placed in the deflectable element is in fluid communication with the irrigation holes of the proximal electrode. The irrigation holes are generally placed radially around, and longitudinally along, the proximal electrode.

In some embodiments, the proximal electrode and the deflectable element define an annular hollow therebetween with the irrigation tube coupled to transfer irrigation fluid into the hollow so that the irrigation tube is in fluid communication with the irrigation holes via the hollow. A pump pumps irrigation fluid from an irrigation reservoir via the irrigation tube into the hollow and out of the irrigation holes.

The ablation power generator is connected to the catheter, and applies an electrical signal between the combined assembly electrode and the proximal electrode to perform radio-frequency (RF) ablation or electroporation of the tissue of the body part.

In some embodiments, the expandable distal end assembly is also irrigated. A second irrigation tube may be placed in the deflectable element and delivers irrigation fluid into a region surrounded by the expandable distal end assembly. In some embodiments, the electrodes of the expandable distal end assembly (e.g., a balloon assembly) include irrigation holes that are in fluid communication with the second irrigation tube. In some embodiments, the irrigation of the expandable distal end assembly and the proximal electrode share the same irrigation tube.

In other embodiments, the proximal electrode is not irrigated. The distal end of the deflectable element and the proximal electrode define an annular region therebetween. Thermally conductive material is placed in the annular region to dissipate heat from the tissue around the proximal electrode thereby preventing or reducing overheating and charring. The thermally conductive material may be formed from a different material than the proximal electrode.

**In** other embodiments, the proximal electrode is formed from a thick piece of thermally conductive material to dissipate heat from the tissue around the proximal electrode thereby preventing or reducing overheating and charring. In some embodiments, the proximal electrode has a maximum thickness measured perpendicular to the axis of the deflectable element of at least 0.05 mm and an inner diameter in the range of about 2 mm to 6 mm.

### SYSTEM DESCRIPTION

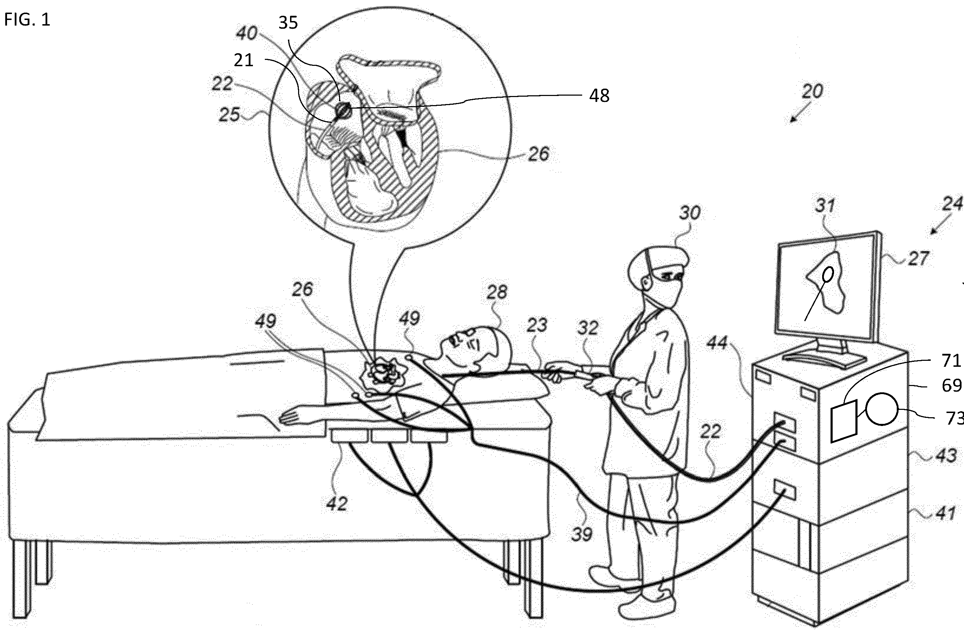

Reference is now made to Fig. 1, which is a schematic view of a medical system 20 constructed and operative in accordance with an exemplary embodiment of the present invention. The system 20 includes a catheter 40 configured to be inserted into a body part of a living subject (e.g., a patient 28). A physician 30 navigates the catheter 40 (for example, a basket catheter produced Biosense Webster, Inc. of Irvine, CA, USA), to a target location in a heart 26 of the patient 28, by manipulating an elongated deflectable element 22 of the catheter 40, using a manipulator 32 near a proximal end of the catheter 40, and/or deflection from a sheath 23. In the pictured embodiment, physician 30 uses catheter 40 to perform electro-anatomical mapping of a cardiac chamber and ablation of cardiac tissue.

Catheter 40 includes an expandable distal end assembly 35 (e.g., a basket assembly), which is inserted in a folded configuration, through sheath 23, and only after the catheter 40 exits sheath 23 does the distal end assembly 35 regain its intended functional shape. By containing distal end assembly 35 in a folded configuration, sheath 23 also serves to minimize vascular trauma on its way to the target location.

Catheter 40 includes a plurality of electrodes 48 disposed on the expandable distal end assembly 35 for sensing electrical activity and/or applying ablation power to ablate tissue of the body part (inset 25). The catheter 40 also includes a proximal electrode 21 disposed on the deflectable element 22 proximal to the expandable distal end assembly 35. Catheter 40 may incorporate a magnetic position sensor (not shown) at the distal edge of deflectable element 22 (i.e., at the proximal edge of the distal end assembly 35). Typically, although not necessarily, the magnetic sensor is a Single-Axis Sensor (SAS). A second magnetic sensor (not shown) may be included at any suitable position on the assembly 35. The second magnetic sensor may be a Triple-Axis Sensor (TAS) or a Dual-Axis Sensor (DAS), or a SAS by way of example only, based for example on sizing considerations. The magnetic sensors, the proximal electrode 21, and electrodes 48 disposed on the assembly 35 are connected by wires running through deflectable element 22 to various driver circuitries in a console 24.

In some embodiments, system 20 comprises a magnetic-sensing subsystem to estimate an ellipticity of the basket assembly 35 of catheter 40, as well as its elongation/retraction state, inside a cardiac chamber of heart 26 by estimating the elongation of the basket assembly 35 from the distance between the magnetic sensors. Patient 28 is placed in a magnetic field generated by a pad containing one or more magnetic field generator coils 42, which are driven by a unit 43. The magnetic fields generated by coil(s) 42 transmit alternating magnetic fields into a region where the body-part is located. The transmitted alternating magnetic fields generate signals in the magnetic sensors, which are indicative of position and/or direction. The generated signals are transmitted to console 24 and become corresponding electrical inputs to processing circuitry 41.

The method of position and/or direction sensing using external magnetic fields and magnetic sensors, is implemented in various medical applications, for example, in the CARTO^{®} system, produced by Biosense-Webster, and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

Processing circuitry 41, typically part of a general-purpose computer, is further connected via a suitable front end and interface circuits 44, to receive signals from body surface-electrodes 49. Processing circuitry 41 is connected to body surface-electrodes 49 by wires running through a cable 39 to the chest of patient 28.

In an embodiment, processing circuitry 41 renders to a display 27, a representation 31 of at least a part of the catheter 40 and a mapped body-part, responsively to computed position coordinates of the catheter 40.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

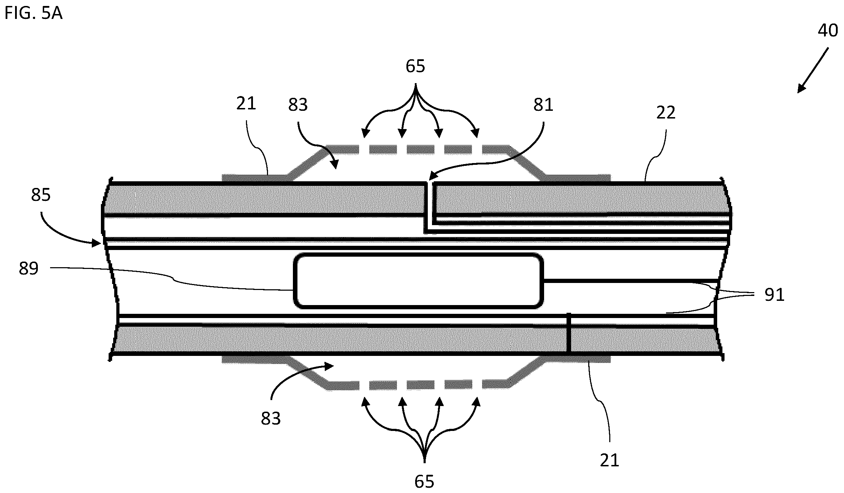

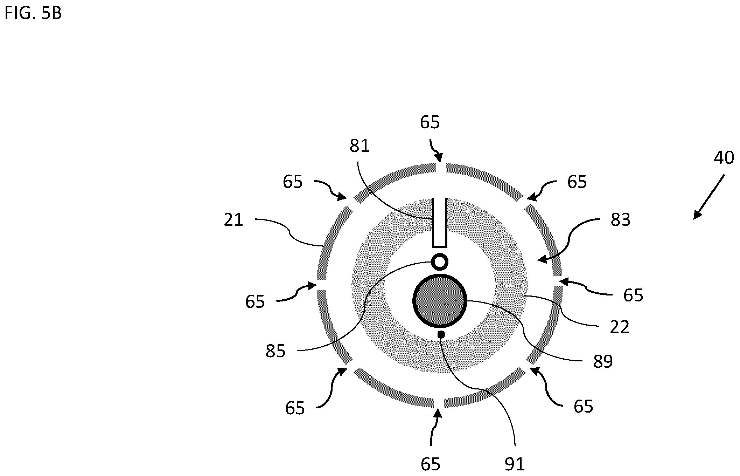

The medical system 20 may also include an ablation power generator 69 (such as an RF signal generator) configured to be connected to the catheter 40, and apply an electrical signal between one or more of the electrodes 48 and the proximal electrode 21. The medical system 20 may also include an irrigation reservoir 71 configured to store irrigation fluid, and a pump 73 configured to be connected to the irrigation reservoir 71 and the catheter 40, and to pump the irrigation fluid from the irrigation reservoir 71 via an irrigation tube through irrigation holes of the catheter 40 as described in more detail with reference to Figs. 5A and 5B.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description. The elements of system 20 and the methods described herein may be further applied, for example, to control an ablation of tissue of heart 26.

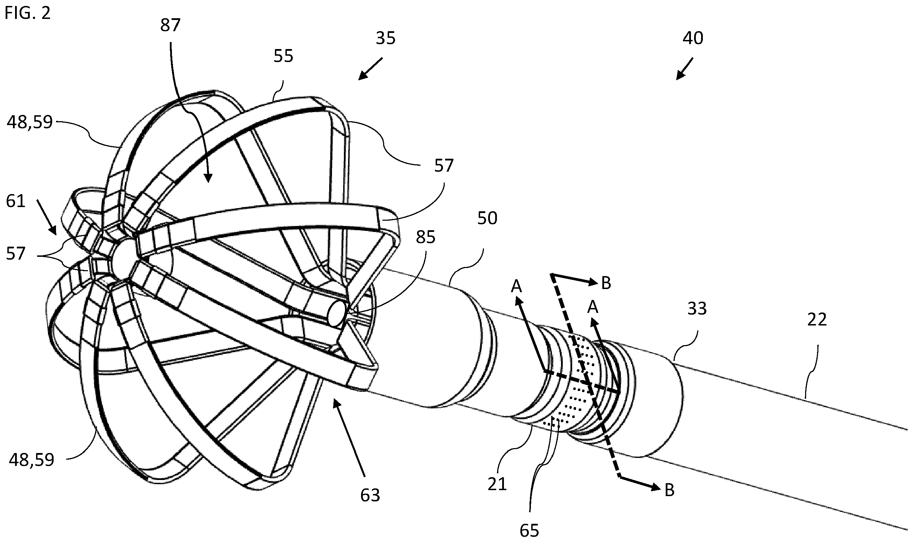

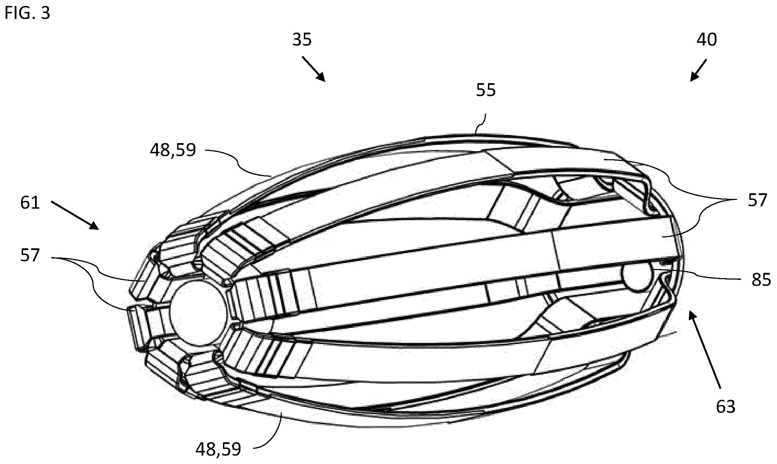

Reference is now made to Figs. 2 and 3. Fig, 2 is a schematic view of the catheter 40 in a deployed form constructed and operative in accordance with an embodiment of the present invention. Fig. 3 is a schematic view of the distal end of the catheter 40 of Fig. 2 in a collapsed form.

The catheter 40 is configured to be inserted into a body part (e.g., the heart 26 (Fig. 1)) of a living subject. The deflectable element 22 of the catheter 40 has a distal end 33. The deflectable element 22 may be produced from any suitable material, for example, polyurethane or polyether block amide. The assembly 35 is disposed distally to the deflectable element 22 and may be connected to the deflectable element 22 via a proximal coupling member 50 at the distal end 33. The proximal coupling member 50 typically comprises a hollow tube and may be formed from any suitable material, for example, but not limited to polycarbonate with or without glass filler, polyether ether ketone (PEEK) with or without glass filler, polyimide, polyamide, or Polyetherimide (PEI) with or without glass filler. The coupling member 50 may formed as an integral part of the deflectable element 22 or as part of the distal end assembly 35 or as a separate element which connects with the deflectable element 22 and the distal end assembly 35.

The assembly 35, which may include a basket assembly, may include multiple splines such as flexible strips 55 (only one labeled for the sake of simplicity) with the electrodes 48 disposed on the splines. In the embodiments of Figs. 2 and 3 each flexible strip 55 includes a single electrode 48 (only some labeled for the sake of simplicity). The assembly 35 may include any suitable number of electrodes 48 with multiple electrodes 48 per strip 55.

In the embodiment of Figs. 2 and 3, each flexible strip 55 is formed of Nitinol which is selectively covered with insulating material (for example, thermoplastic polymer resin shrink wrap (PET)) in the distal and proximal regions 57 (only some labeled for the sake of simplicity) of the flexible strips 55 leaving a central region 59 (only some labeled for the sake of simplicity) of the flexible strips 55 as an electrically active region to perform mapping and/or perform ablation or electroporation, by way of example. The structure of the assembly 35 may vary. For example, flexible strips 55 (or other splines) may include flexible printed circuit boards (PCBs), or a shape-memory alloy such as Nitinol. The electrically active region of each flexible strip 55 may be larger or smaller than that shown in Fig. 2, and/or more centrally or proximally disposed on each flexible strip 55.

Embodiments described herein refer mainly to a basket distal-end assembly 35, purely by way of example. In alternative embodiments, the disclosed techniques can be used with any other suitable type of distal-end assembly.

The distal end assembly 35 includes a distal portion 61, and a proximal portion 63, and is configured to expand from a collapsed form (shown in Fig. 3) to an expanded deployed form (shown in Fig. 2). The relaxed state of the distal end assembly 35 is the expanded deployed form shown in Fig. 2. The distal end assembly 35 is configured to collapse into the collapsed form when the catheter 40 is retracted in a sheath 23 (Fig. 1) and is configured to expand to the expanded deployed form when the catheter 40 is removed from the sheath 23. The relaxed shape of the distal end assembly 35 may be set by forming the flexible strips 55 from any suitable resilient material such as Nitinol or PEI. In some embodiments, the relaxed state of the expandable distal end assembly 35 may be the collapsed form, and the expandable distal end assembly 35 is expanded using a pull wire or element connected to the distal portion 61 and fed through a lumen in the deflectable element 22.

The proximal electrode 21 is disposed at the distal end 33 of the deflectable element 22 proximally to the expandable distal end assembly 35, and generally extends circumferentially around the deflectable element 22. The proximal electrode 21 includes irrigation holes 65 (only some labeled for the sake of simplicity) through which to irrigate the body part. The irrigation holes 65 are generally disposed radially around, and/or longitudinally along, the proximal electrode 21. The irrigation holes may have any suitable diameter, for example, in the range of 25 to 100 microns. The holes may be formed using any suitable technique, for example, laser drilling or electrical discharge machining (EDM). The proximal electrode 21 may include any suitable number of holes, for example, in the range of 4 to 100 holes. In one example, the proximal electrode 21 includes 5 proximally disposed holes and 5 distally disposed holes. An additional irrigation tube 85 is disposed in element 22 as explained in greater detail subsequently.

The ablation power generator 69 (Fig. 1) is configured to be connected to the catheter 40, and apply an electrical signal between at least one of the electrodes 48 and the proximal electrode 21. In some embodiments, the ablation power generator 69 is configured to apply the electrical signal between at least one of the electrodes 48 and the proximal electrode 21 to perform electroporation of tissue of the body part.

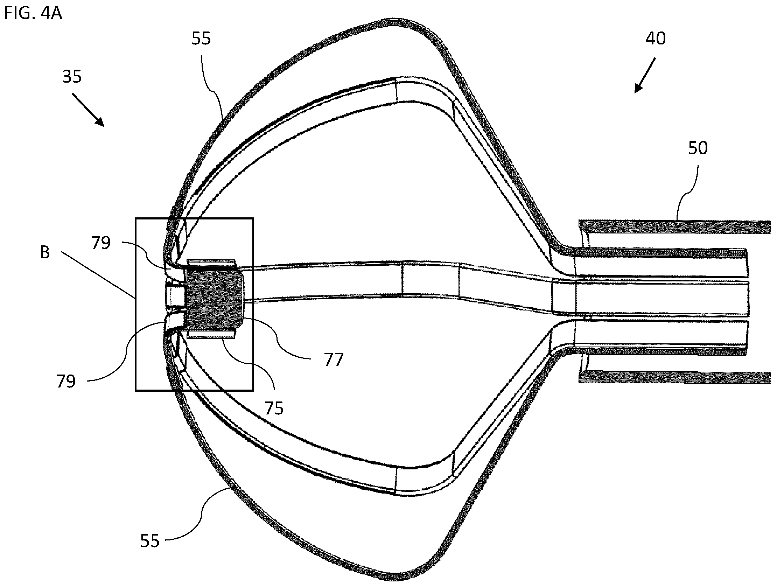

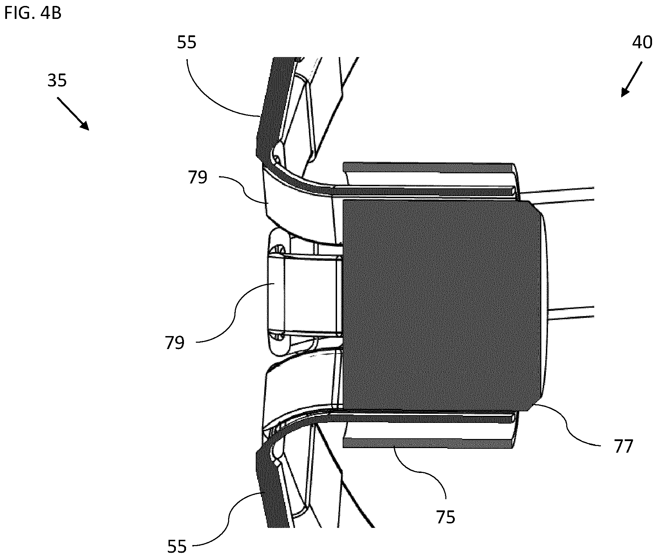

Reference is now made to Figs. 4A and 4B. Fig. 4A is a cross-sectional view of the distal end of the catheter 40 of Fig. 2. Fig. 4B is a more detailed cross-sectional view of the distal end of the catheter 40 inside block B of Fig. 4A.

The distal ends of the flexible strips 55 (only two labeled for the sake of simplicity) are folded over and connected to a distal connector 75, which in some embodiments is a tube (e.g., polymer tube) or slug (e.g., polymer slug). The distal connector 75 may be formed from any suitable material, for example, but not limited to polycarbonate with or without glass filler, PEEK with or without glass filler, or PEI with or without glass filler. In some embodiments, the flexible strips 55 may be connected to the distal connector 75 without being folded over so that when the distal end assembly 35 is collapsed the flexible strips 55 are approaching a flat formation along their length. The proximal ends of the flexible strips 55 are connected to the proximal coupling member 50. The flexible strips 55 may be connected to the distal connector 75 and the proximal coupling member 50 using a suitable adhesive, such as an epoxy adhesive.

In some embodiments, the catheter 40 includes a nose cap 77 inserted into the distal connector 75. The nose cap 77 may be used to help secure the flexible strips 55 to the distal connector 75. The nose cap 77 may be formed from any suitable material, for example, but not limited to polycarbonate with or without glass filler, PEEK with or without glass filler, or PEI with or without glass filler. The nose cap 77 may optionally be sized to provide a pressure fit against the flexible strips 55 to prevent the flexible strips 55 from being pulled away from the inner surface of the distal connector 75.

In some embodiments, the thickness of the distal portions of the flexible strips 55 may be reduced (compared to the rest of the flexible strips 55) to create hinges 79 (one hinge 79 per flexible strip 55) to allow the flexible strips 55 to bend sufficiently between the collapsed form and the deployed expanded form of the expandable distal end assembly 35. Only two of the hinges 79 are labeled for the sake of simplicity. The hinges 79 of the flexible strips 55 may be reinforced using a flexible material such as a yarn (not shown). The hinges 79 (including the yarn and covering layers) may have any suitable thickness, for example, in the range of about 10 to 140 microns. The yarn may comprise any one or more of the following: an ultra-high-molecular-weight polyethylene yarn; or a yarn spun from a liquid-crystal polymer. The yarn may be any suitable linear density, for example, in a range between about 25 denier and 250 denier.

Reference is now made to Figs. 5A and 5B. Fig 5A is a cross-sectional view of the catheter 40 of Fig. 2 along line A:A. Fig. 5B is a cross-sectional view of the catheter of Fig. 2 along line B:B.

Figs. 5A and 5B show the proximal electrode 21 which extends circumferentially around the deflectable element 22. The edges of the proximal electrode 21 may be connected to the deflectable element 22 using a suitable adhesive and/or using a covering such as a thermoplastic polymer resin shrink wrap. Figs. 5A and 5B show some of the irrigation holes 65 (only some labeled for the sake of simplicity) in the proximal electrode 21. The proximal electrode 21 may have any suitable length measured parallel to the direction of elongation of the deflectable element 22, for example, in the range of about 2 and 10 mm.

The catheter 40 includes an irrigation tube 81 disposed in the deflectable element 22 and configured to be in fluid communication with the irrigation holes 65 of the proximal electrode 21. The pump 73 (Fig. 1) is configured to be connected to the irrigation reservoir 71 (Fig. 1) and the catheter 40, and to pump the irrigation fluid from the irrigation reservoir 71 through the irrigation holes 65 via the irrigation tube 81.

The inner surface of the proximal electrode 21 and the deflectable element 22 define an annular hollow 83 therebetween. The irrigation tube 81 is coupled to the annular hollow 83 to transfer the irrigation fluid into the hollow 83. The irrigation tube 81 is generally disposed on the other side of the annular hollow 83 to the irrigation holes 65. Therefore, the irrigation tube 81 is in fluid communication with the irrigation holes 65 via the hollow 83. The pump 73 (Fig. 1) is configured to pump the irrigation fluid from the irrigation reservoir 71 via the irrigation tube 81 into the hollow 83 and out of the irrigation holes 65. The collection of the irrigation fluid in the annular hollow 83 acts to cool the outer surface of the proximal electrode 21 and not just the portions close to the irrigation holes 65.

The catheter 40 may include another irrigation tube 85 disposed in the deflectable element 22 and configured to deliver irrigation fluid into a region 87 (Fig. 2) surrounded by the flexible strips 55 of the expandable distal end assembly 35. The irrigation tube 85 typically extends into the expandable distal end assembly 35 as shown in Figs. 2 and 3.

In some embodiments, the catheter 40 includes a position sensor 89 (such as a magnetic position sensor) disposed in the deflectable element 22. Figs. 5A and 5B also show wires 91 disposed therein connecting the electrodes 48, the proximal electrode 21, and the position sensor 89 with the proximal end of the catheter 40.

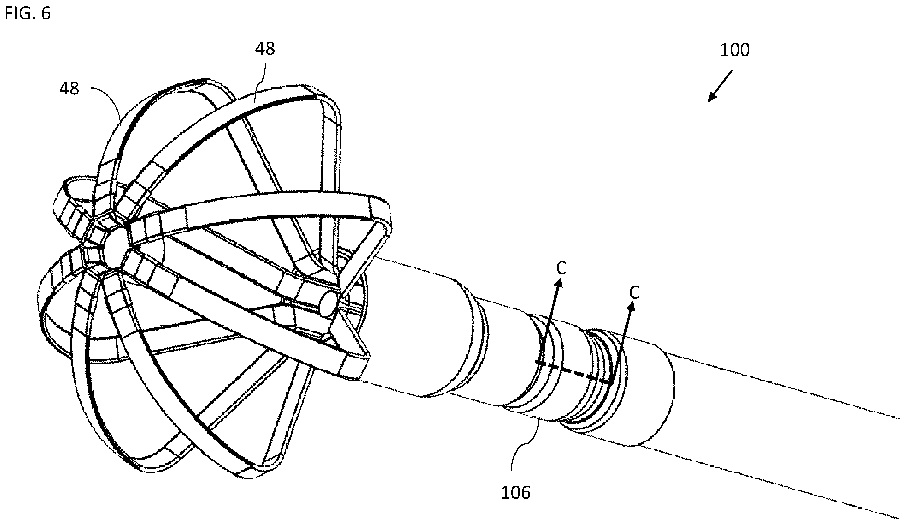

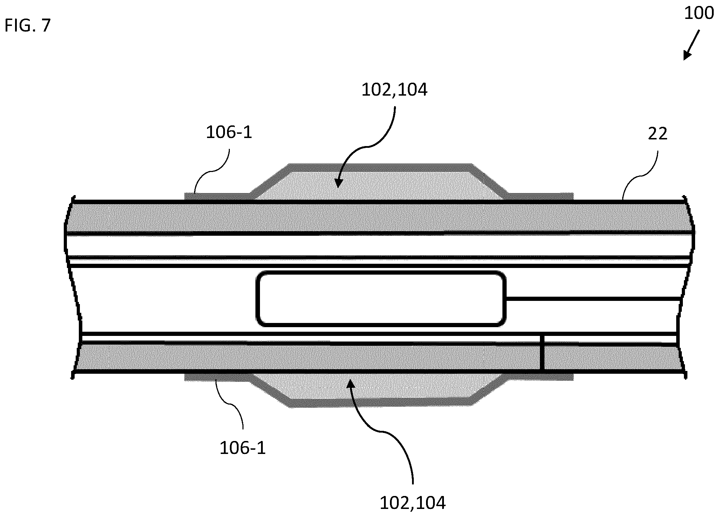

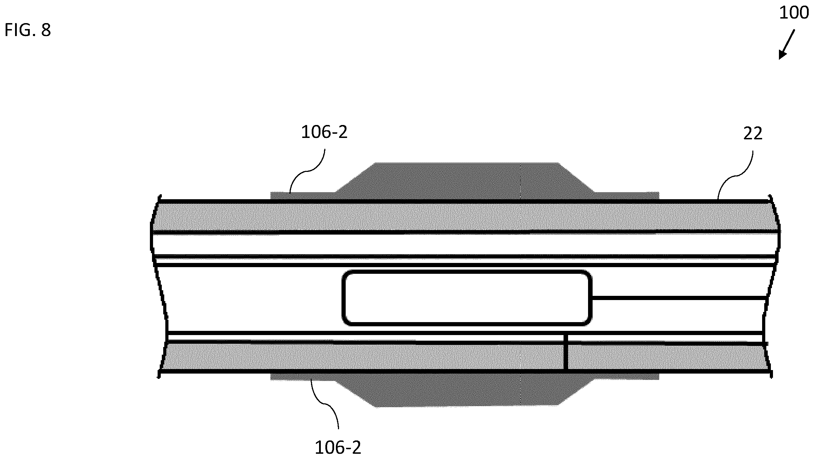

Reference is now made to Fig. 6, which is a schematic view of a catheter 100 in a deployed form constructed and operative in accordance with an alternative embodiment of the present invention. The catheter 100 is substantially the same as the catheter 40 of Figs. 2 and 3 except for the following differences. The catheter 100 includes a proximal electrode 106, which is not irrigated. The proximal electrode 106 may either be cooled by filling with a thermally conductive material as described with reference to a proximal electrode 106-1 of Fig. 7, or by forming the proximal electrode from a thermally conductive material of sufficient thickness to dissipate heat as described with reference to a proximal electrode 106-2 of Fig. 8.

Reference is now made to Fig. 7, which is a cross-sectional view of the catheter 100 of Fig. 6 along line C:C. The proximal electrode 106-1 and the distal end of the deflectable element 22 define an annular region 102 therebetween. The catheter 100 includes thermally conductive material 104, which is disposed in the annular region 102, generally, but not necessarily, filling the annular region 102, and generally in contact with at least part of the inner surface of the proximal electrode 106-1. The thermally conductive material 104 may be formed from a different material than the proximal electrode 106-1.

The term "thermally conductive material", as used in the specification and claims, is defined as a material with a thermal conductivity greater than or equal to 1 Watt per meter Kelvin (W/mK) at 25 degrees Centigrade. The thermally conductive material 104 may be any suitable thermally conductive material, for example, but not limited to, platinum, palladium, gold, or thermally conductive epoxy. In some embodiments, the thermally conductive material 104 is first wrapped around the outer surface of the deflectable element 22, and then the proximal electrode 106-1 is wrapped around the thermally conductive material 104. In other embodiments, the proximal electrode 106-1 is first fixed around the deflectable element 22 (as a single piece or from two halves subsequently joined together) and then the thermally conductive material 104 is injected below the proximal electrode 106-1 through a hole (not shown) in the proximal electrode 106-1.

The wall thickness of the proximal electrode 106-1 may have any suitable value, for example, in the range of about 0.01mm to 0.25mm. The thickness of the thermally conductive material 104 may have any suitable value, for example, in the range of about 0.01mm to 0.25mm. The proximal electrode 106-1 may have any suitable length measured parallel to the direction of elongation of the deflectable element 22, for example, between about 2 mm and 10 mm.

It should be noted that the irrigation tube 81 (Figs. 5A and 5B) is not included within the deflectable element 22 shown in Fig. 7.

Reference is now made to Fig. 8, which is a cross-sectional view of the catheter 100 of Fig. 6 along line C:C constructed and operative in accordance with another alternative embodiment of the present invention. The proximal electrode 106-2 shown in Fig. 8 has a wall thickness which is greater than the wall thickness of the proximal electrode 106-1 described with reference to Fig. 7.

The proximal electrode 106-2 may have any suitable wall thickness. In some embodiments, the proximal electrode 106-2 may have a maximum thickness measured perpendicular to the axis of the deflectable element 22 of at least 0.05 mm and an inner diameter in the range of 2 mm to 6 mm.

The proximal electrode 106-2 may have any suitable length measured parallel to the direction of elongation of the deflectable element 22 of between about 2 mm and 10 mm.

The proximal electrode 106-2 is formed from a thermally conductive material, which provides dissipation of heat formed during electroporation and/or RF ablation. The thermally conductive material may be any suitable thermally conductive material, for example, but not limited to, platinum, palladium, or gold.

The proximal electrode 106-2 may each be formed as a flat electrode which is wound around the outer surface of the deflectable element 22 to form a ring or as two half rings which are connected together around the deflectable element 22.

Each proximal electrode 106-2, 106-1 (Fig. 7), 21 (Figs. 5A and 5B), has a non-uniform surface, which bulges away from the outer surface of the deflectable element 22. The proximal electrodes may have any suitable shape. For example, the proximal electrode 21, 106-1, 106-2 may be formed as ring having a uniform outer diameter along the length of the proximal electrode 21.

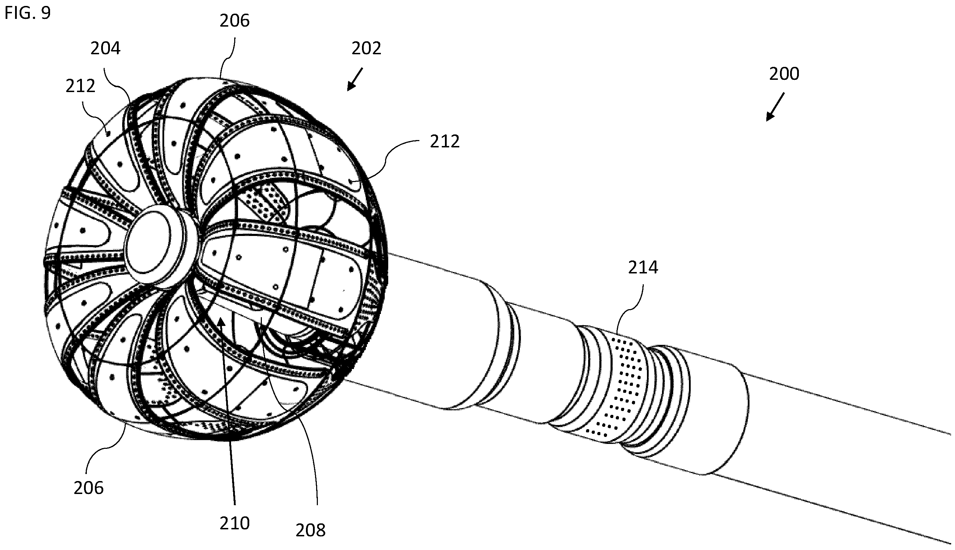

Reference is now made to Fig. 9, which is a schematic view of a balloon catheter 200 in a deployed form constructed and operative in accordance with yet another alternative embodiment of the present invention. The catheter 200 is substantially the same as the catheter 40 of Fig. 2 except that the catheter 200 includes an expandable distal end assembly 202, which includes an inflatable balloon 204 with the electrodes 206 (only some labeled for the sake of simplicity) disposed thereon. The catheter 200 includes an irrigation tube 208 which is disposed in the deflectable element 22 and extends into a region 210 surrounded by the inflatable balloon 204. The electrodes 206 of the expandable distal end assembly 202 include irrigation holes 212 (only some labeled for the sake of simplicity) that are in fluid communication with the irrigation tube 208. The catheter 200 includes a proximal electrode 214 in substantially the same form as the proximal electrode 21 described with reference to Figs. 5A and 5B. In some embodiments, the proximal electrode 214 may be replaced with the proximal electrode 106-1 of Fig. 7 or with the proximal electrode 106-2 of Fig. 8.

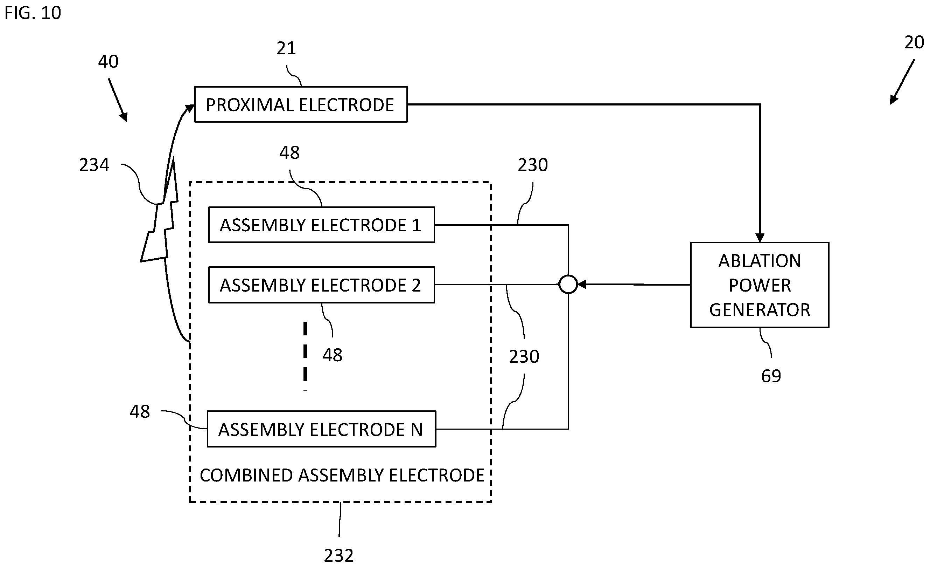

Reference is now made to Fig. 10, which is a schematic view of electrode connections in the catheter 40 of medical system 20 constructed and operative in accordance with an exemplary embodiment of the present invention.

The catheter 40 may include one or more electrical connections 230 configured to electrically connect together at least two (and optionally all) of the assembly electrodes 48 to act as a combined assembly electrode 232.

In some embodiments, the electrical connection(s) 230 may be configured to selectively connect together the assembly electrodes 48 to act as the combined assembly electrode 232 and also allow the electrodes 48 to act as individual electrodes, for example, for sensing positions, electrical activations, and performing individual ablation. In such embodiments, the electrical connections 230 may include switching circuitry (not shown) which enables selectively connecting together two or more (and optionally all) of the assembly electrodes 48.

In other embodiments, the electrical connection(s) 230 permanently electrically connects together the at least two (and optionally all) of the assembly electrodes 48 to act as the combined assembly electrode 232.

The ablation power generator 69 is configured to be connected to the catheter 40, and apply an electrical signal (arrow 234) to the combined assembly electrode 232 so as to ablate tissue of the body part. In some embodiments, the ablation power generator 69 is configured to apply the electrical signal 234 to the combined assembly electrode 48 so as to perform electroporation of tissue of the body part.

The electrical signal 234 is generally applied between the combined assembly electrode 232 and a return electrode. The return electrode may be located in any suitable location, for example, on the catheter 40, as an indifferent electrode attached to the patient's skin or on another catheter. In some embodiments, the proximal electrode 21 acts as the return electrode.

Therefore, in some embodiments, the ablation power generator 69 is configured to apply an electrical signal between the combined assembly electrode 232 and the proximal electrode 21. In some embodiments, the ablation power generator 69 is configured to apply the electrical signal between the combined assembly electrode 232 and the proximal electrode 21 to perform electroporation of tissue of the body part.

As previously mentioned, the ablation may lead to excessive heating in the region of the proximal electrode 21. Therefore, the proximal electrode 21 may apply cooling to surrounding tissue using irrigation as described above with reference to Figs. 2, 5A and 5B.

The electrical connections 230 may be implemented with other catheters to connect assembly electrodes together to form a combined assembly electrode.

In some embodiments, two or more (and optionally all) of the assembly electrodes 48 (Fig. 6) of the catheter 100 may be connected (selectively or permanently) using the electrical connections 230. The proximal electrode 106 (Fig. 6) or any other suitable electrode may act as the return electrode. The proximal electrode 106 may provide cooling using the thermally conductive material 104 disposed in the annular region 102 (Fig. 7) of the proximal electrode 106, as described in more detail above with reference to Fig. 7. Alternatively, the proximal electrode 106 may provide cooling by forming the proximal electrode 106 (Fig. 8) from a thermally conductive material having a maximum thickness measured perpendicular to the axis of the deflectable element of at least 0.05 mm and an inner diameter in the range of 2 mm to 6 mm, as described in more detail above with reference to Fig. 8.

In some embodiments, two or more (and optionally all) of the electrodes 206 of the expandable distal end assembly 202 of the catheter 200 of Fig. 9 may be connected (selectively or permanently) using the electrical connections 230. The proximal electrode 214 (Fig. 9) or any other suitable electrode may act as the return electrode.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove but is only limited by the scope of the appended claims.

## Claims

1. A medical system (20) comprising a catheter (40) configured to be inserted into a body part of a living subject, and including:
a deflectable element (22) having a distal end (33);
an expandable distal end assembly (35) disposed at the distal end of the deflectable element, and comprising a plurality of assembly electrodes (48), and configured to expand from a collapsed form to an expanded deployed form;
a proximal electrode (21) disposed at the distal end of the deflectable element proximally to the expandable distal end assembly, and extending circumferentially around the deflectable element;
at least one electrical connection (230) configured to electrically connect together at least two of the assembly electrodes to act as a combined assembly electrode; and
an ablation power generator (69) configured to be connected to the catheter, and apply an electrical signal between the combined assembly electrode and a selected electrode,
**characterized in that** the proximal electrode and the distal end of the deflectable element define an annular region (102) therebetween, the catheter also including thermally conductive material (104) disposed in the annular region, the thermally conductive material being formed from a different material than the proximal electrode.

2. The system according to claim 1, wherein the selected electrode is the proximal electrode.

3. The system according to claim 1, wherein the at least one electrical connection permanently electrically connects together the at least two assembly electrodes to act as the combined assembly electrode.

4. The system according to claim 1, wherein the proximal electrode includes irrigation holes (65) through which to irrigate the body part, the catheter also including an irrigation tube (81) disposed in the deflectable element and configured to be in fluid communication with the irrigation holes of the proximal electrode.

5. The system according to claim 4, wherein the irrigation holes are disposed radially around the proximal electrode, or are disposed longitudinally along the proximal electrode.

6. The system according to claim 4, wherein the proximal electrode and the deflectable element define an annular hollow (83) therebetween, the irrigation tube being coupled to transfer irrigation fluid into the hollow, the irrigation tube being in fluid communication with the irrigation holes via the hollow.

7. The system according to claim 4, further comprising:
an irrigation reservoir (71) configured to store irrigation fluid; and
a pump (73) configured to be connected to the irrigation reservoir and the catheter, and to pump the irrigation fluid from the irrigation reservoir through the irrigation holes via the irrigation tube.

8. The system according to claim 1, wherein the ablation power generator is configured to apply the electrical signal between the combined assembly electrode and the proximal electrode to perform electroporation of tissue of the body part.

9. The system according to claim 1, further comprising an irrigation tube (85) disposed in the deflectable element and configured to deliver irrigation fluid into a region surrounded by the expandable distal end assembly.

10. The system according to claim 1, wherein the proximal electrode has a maximum thickness measured perpendicular to the axis of the deflectable element of at least 0.05 mm and an inner diameter in the range of 2 mm to 6 mm.

11. The system according to claim 1, wherein the at least one electrical connection permanently electrically connects together all of the assembly electrodes to act as the combined assembly electrode.

12. The system according to claim 1, wherein the expandable distal end assembly includes at least one of: an expandable basket comprising a plurality of splines (55), the electrodes being disposed on the splines; or an inflatable balloon (204) with the electrodes disposed thereon.

## Patentansprüche

1. Medizinisches System (20), umfassend einen Katheter (40), der konfiguriert ist, um in einen Körperteil eines lebenden Subjekts eingeführt zu werden, und einschließlich:
eines ablenkbaren Elements (22), das ein distales Ende (33) aufweist;
einer erweiterbaren distalen Endanordnung (35), die an dem distalen Ende des ablenkbaren Elements eingerichtet ist und eine Vielzahl von Anordnungselektroden (48) umfasst und konfiguriert ist, um sich von einer zusammengeklappten Form in eine ausgedehnte entfaltete Form zu erweitern;
einer proximalen Elektrode (21), die an dem distalen Ende des auslenkbaren Elements proximal zu der erweiterbaren distalen Endanordnung eingerichtet ist und sich kreisförmig um das auslenkbare Element herum erstreckt;
mindestens einer elektrischen Verbindung (230), die konfiguriert ist, um mindestens zwei der Anordnungselektroden miteinander elektrisch zu verbinden, um als eine kombinierte Anordnungselektrode zu fungieren; und
eines Ablationsstromgenerators (69), der konfiguriert ist, um mit dem Katheter verbunden zu sein und ein elektrisches Signal zwischen der kombinierten Anordnungselektrode und einer ausgewählten Elektrode anzulegen,
**dadurch gekennzeichnet, dass** die proximale Elektrode und das distale Ende des ablenkbaren Elements dazwischen einen ringförmigen Bereich (102) definieren, wobei der Katheter außerdem wärmeleitendes Material (104) einschließt, das in dem ringförmigen Bereich eingerichtet ist, wobei das wärmeleitende Material aus einem anderen Material als die proximale Elektrode ausgebildet ist.

2. System nach Anspruch 1, wobei die ausgewählte Elektrode die proximale Elektrode ist.

3. System nach Anspruch 1, wobei die mindestens eine elektrische Verbindung die mindestens zwei Anordnungselektroden dauerhaft elektrisch miteinander verbindet, um als die kombinierte Anordnungselektrode zu fungieren.

4. System nach Anspruch 1, wobei die proximale Elektrode Spüllöcher (65) einschließt, durch die der Körperteil gespült werden soll, wobei der Katheter außerdem einen Spülschlauch (81) einschließt, der in dem ablenkbaren Element eingerichtet und konfiguriert ist, um in Fluidkommunikation mit den Spüllöchern der proximalen Elektrode zu stehen.

5. System nach Anspruch 4, wobei die Spüllöcher radial um die proximale Elektrode herum oder in Längsrichtung entlang der proximalen Elektrode eingerichtet sind.

6. System nach Anspruch 4, wobei die proximale Elektrode und das ablenkbare Element einen ringförmigen Hohlraum (83) dazwischen definieren, wobei der Spülschlauch gekoppelt ist, um Spülfluid in den Hohlraum zu leiten, wobei der Spülschlauch über den Hohlraum in Fluidkommunikation mit den Spüllöchern steht.

7. System nach Anspruch 4, ferner umfassend:
ein Spülreservoir (71), das konfiguriert ist, um Spülfluid zu lagern; und
eine Pumpe (73), die konfiguriert ist, um mit dem Spülreservoir und dem Katheter verbunden zu sein und um Spülfluid aus dem Spülreservoir über den Spülschlauch durch die Spüllöcher zu pumpen.

8. System nach Anspruch 1, wobei der Ablationsstromgenerator konfiguriert ist, um das elektrische Signal zwischen der kombinierten Anordnungselektrode und der proximalen Elektrode anzulegen, um eine Elektroporation von Gewebe des Körperteils durchzuführen.

9. System nach Anspruch 1, ferner umfassend einen Spülschlauch (85), der in dem ablenkbaren Element eingerichtet und konfiguriert ist, um Spülfluid in einen Bereich abzugeben, der von der erweiterbaren distalen Endanordnung umgeben ist.

10. System nach Anspruch 1, wobei die proximale Elektrode eine maximale Dicke, die senkrecht zu der Achse des auslenkbaren Elements gemessen wird, von mindestens 0,05 mm und einen Innendurchmesser im Bereich von 2 mm bis 6 mm aufweist.

11. System nach Anspruch 1, wobei die mindestens eine elektrische Verbindung alle Anordnungselektroden dauerhaft elektrisch miteinander verbindet, um als die kombinierte Anordnungselektrode zu fungieren.

12. System nach Anspruch 1, wobei die erweiterbare distale Endanordnung mindestens eines einschließt von: einem erweiterbaren Korb, umfassend eine Vielzahl von Keilen (55), wobei die Elektroden auf den Keilen eingerichtet sind; oder einem aufblasbaren Ballon (204) mit darauf eingerichteten Elektroden.

## Revendications

1. Système médical (20) comprenant un cathéter (40) conçu pour être inséré dans une partie de corps d'un sujet vivant, et comportant :
un élément pouvant fléchir (22) ayant une extrémité distale (33) ;
un ensemble d'extrémité distale expansible (35) disposé au niveau de l'extrémité distale de l'élément pouvant fléchir, et comprenant une pluralité d'électrodes d'ensemble (48), et conçu pour s'expanser d'une forme rétractée à une forme déployée expansée ;
une électrode proximale (21) disposée au niveau de l'extrémité distale de l'élément pouvant fléchir, proximalement à l'ensemble d'extrémité distale expansible, et s'étendant circonférentiellement autour de l'élément pouvant fléchir ;
au moins une connexion électrique (230) configurée pour connecter électriquement au moins deux des électrodes d'ensemble pour jouer le rôle d'électrode d'ensemble combinée ; et
un générateur de puissance d'ablation (69) configuré pour être connecté au cathéter, et appliquer un signal électrique entre l'électrode d'ensemble combinée et une électrode sélectionnée,
**caractérisé en ce que** l'électrode proximale et l'extrémité distale de l'élément pouvant fléchir définissent une région annulaire (102) entre elles, le cathéter comportant également un matériau thermoconducteur (104) disposé dans la région annulaire, le matériau thermoconducteur étant formé d'un matériau différent de celui de l'électrode proximale.

2. Système selon la revendication 1, dans lequel l'électrode sélectionnée est l'électrode proximale.

3. Système selon la revendication 1, dans lequel l'au moins une connexion électrique connecte électriquement de manière permanente les au moins deux électrodes d'ensemble pour jouer le rôle d'électrode d'ensemble combinée.

4. Système selon la revendication 1, dans lequel l'électrode proximale comporte des trous d'irrigation (65) par le biais desquels on irrigue la partie de corps, le cathéter comportant également un tube d'irrigation (81) disposé dans l'élément pouvant fléchir et conçu pour être en communication fluidique avec les trous d'irrigation de l'électrode proximale.

5. Système selon la revendication 4, dans lequel les trous d'irrigation sont disposés radialement autour de l'électrode proximale, ou sont disposés longitudinalement le long de l'électrode proximale.

6. Système selon la revendication 4, dans lequel l'électrode proximale et l'élément pouvant fléchir définissent un creux annulaire (83) entre eux, le tube d'irrigation étant accouplé pour transférer un fluide d'irrigation dans le creux, le tube d'irrigation étant en communication fluidique avec les trous d'irrigation par l'intermédiaire du creux.

7. Système selon la revendication 4, comprenant en outre :
un réservoir d'irrigation (71) conçu pour stocker un fluide d'irrigation ; et
une pompe (73) conçue pour être raccordée au réservoir d'irrigation et au cathéter, et pour pomper le fluide d'irrigation du réservoir d'irrigation à travers les trous d'irrigation par l'intermédiaire du tube d'irrigation.

8. Système selon la revendication 1, dans lequel le générateur de puissance d'ablation est configuré pour appliquer le signal électrique entre l'électrode d'ensemble combinée et l'électrode proximale pour mettre en œuvre une électroporation de tissu de la partie de corps.

9. Système selon la revendication 1, comprenant en outre un tube d'irrigation (85) disposé dans l'élément pouvant fléchir et conçu pour délivrer un fluide d'irrigation dans une région entourée par l'ensemble d'extrémité distale expansible.

10. Système selon la revendication 1, dans lequel l'électrode proximale a une épaisseur maximale mesurée perpendiculaire à l'axe de l'élément pouvant fléchir d'au moins 0,05 mm et un diamètre interne dans la plage de 2 mm à 6 mm.

11. Système selon la revendication 1, dans lequel l'au moins une connexion électrique connecte électriquement de manière permanente toutes les électrodes d'ensemble pourr jouer le rôle d'électrode d'ensemble combinée.

12. Système selon la revendication 1, dans lequel l'ensemble d'extrémité distale expansible comporte au moins l'un parmi un panier expansible comprenant une pluralité de cannelures (55), les électrodes étant disposées sur les cannelures ; ou un ballonnet gonflable (204) avec les électrodes disposées sur celui-ci.
